# EUROPÄISCHE PATENTSCHRIFT

(11) **EP 2 352 417 B1**
(45) Veröffentlichungstag und Bekanntmachung des Hinweises auf die Patenterteilung: **13.11.2013**
(21) Anmeldenummer: 09778676.8
(22) Anmeldetag: 23.09.2009
(51) Int. Cl.: A61B 5/024, F16H 59/02, A61B 5/1455

(54) **VORRICHTUNG ZUM ERFASSEN UND ÜBERWACHEN ZUMINDEST EINES VITALPARAMETERS EINER PERSON IN EINEM KRAFTFAHRZEUG**
APPARATUS FOR RECORDING AND MONITORING AT LEAST ONE VITAL SIGN OF A PERSON IN A MOTOR VEHICLE
DISPOSITIF POUR DETECTER ET SURVEILLER AU MOINS UN PARAMETRE VITAL D'UNE PERSONNE DANS UN VEHICULE MOTORISE

(30) Priorität: 07.10.2008 DE 102008050639; 06.11.2008 DE 102008056250
(43) Veröffentlichungstag der Anmeldung: 10.08.2011
(73) Patentinhaber: Fraunhofer-Gesellschaft zur Förderung der angewandten Forschung e.V., 80686 München (DE); ZF Friedrichshafen AG, 88038 Friedrichshafen (DE)
(72) Erfinder: COURONNÉ, Robert, 91058 Erlangen (DE); MOERSDORF, Hans-Joachim, 90763 Fürth (DE); TOBOLA, Andreas, 91334 Hemhofen (DE); ERSHOV, Sergey, 91054 Erlangen (DE); GASSMANN, Hans-Josef, 31715 Meerbeck (DE); HAEVESCHER, Rainer, 32351 Stemwede (DE); RAKE, Ludger, 49356 Diepholz (DE); MEYER, Joerg, 49419 Wagenfeld (DE)
(74) Vertreter: Stöckeler, Ferdinand
(86) Internationale Anmeldenummer: PCT/EP2009/006876
(87) Internationale Veröffentlichungsnummer: WO 2010/040452

(56) Entgegenhaltungen:
- EP-A- 1 632 371
- WO-A1-96/13208
- CA-A1- 2 517 184
- DE-U1- 29 901 849
- US-A1- 2003 036 685
- US-A1- 2005 239 075
- US-A1- 2006 009 685
- HUG R: "LE POULS AU VOLANT" 14. April 2005 (2005-04-14), REVUE AUTOMOBILE, BUECHLER GRAFINO AG, BERNE, CH, PAGE(S) 17 , XP001226488 ISSN: 0035-0761 das ganze Dokument

## Beschreibung

Die vorliegende Anmeldung bezieht sich auf eine Vorrichtung zum Erfassen und/oder Überwachen zumindest eines Vitalparameters einer Person, z. B. in einem Kraftfahrzeug.

Bekannte Verfahren zum Erfassen von Vitalparametern sind beispielsweise die optische Plethysmographie und Pulsoxymetrie. Dabei basieren die optische Plethysmographie und Pulsoxymetrie grundsätzlich auf den gleichen Messverfahren und stellen eine Methode zur nicht-invasiven Ermittlung der Pulsrate, Pulsratenvariabilität und arteriellen Sauerstoffsättigung über die Messung der Lichtabsorption bzw. der Lichtremission im Gewebe dar. Dabei basiert die optische Plethysmographie auf der durch Volumenschwankungen von Körperflüssigkeiten in Gefäßen, z.B. Blut, bedingten Änderung der Lichtabsorption, während die Pulsoxymetrie auf der unterschiedlichen Lichtabsorption bzw. Lichtremission eines roten und eines infraroten Lichtstrahls bei Durchleuchtung der Haut und des Gewebes basiert.

Die optische Plethysmographie und die Pulsoxymetrie sind Bestandteil des klinischen Alltags geworden und werden sowohl für die Standardüberwachung von Patienten als auch für diagnostische Zwecke verwendet. Unter anderem wird die Pulsoxymetrie immer mehr für "Homecare", d. h. für die Überwachung bzw. "Monitoring" des Patienten in der häuslichen Umgebung, eingesetzt. Die Hauptanwendungen sind hierbei: a) Betreuung der Patienten mit kardiologischen Risikofaktoren, b) Diagnostik von Schlafstörungen, c) Müdigkeitserkennung, d) Stresserkennung, und e) chronische Lungenerkrankungen. Bei der Pulsoxymetrie werden standardmäßig die SpO₂-Werte über einen optischen Sensor am Finger, Zeh oder Ohrläppchen abgenommen. Die Messung erfolgt typischerweise mit einem Clipsensor oder einem Klebesensor. Um die pulsoxymetrischen Messungen nicht nur im Krankenhaus durchführen zu können, bekommt der Patient typischerweise ein mobiles Gerät, dessen Anwendungsteil bzw. Sensor direkt an dem Körper angebracht wird. Dies begrenzt die Bewegungsfreiheit der Patienten und ist als Lösung in einem Automobil aufgrund der Einschränkung der Bewegungsfreiheit zum großen Teil nicht akzeptabel.

Teilweise wurde vorgeschlagen, optische Sensoren für die zuvor genannten Messverfahren in den Lenkradkranz zu integrieren. Hier hat sich jedoch häufig die schlechte Signalqualität der optischen Messung als ein Problem herausgestellt.

Die Veröffentlichung EP 1 632 371 A1 beschreibt eine Klimaanlage für ein Fahrzeug, bei der eine Sauerstoffsättigung des Fahrers anhand einer Lichttransmissionsmethode oder einer Lichtreflexionsmethode, die an einem Finger des Fahrers durchgeführt wird, gemessen wird. In einem Beispiel für die Lichttransmissionsmessung ist eine Messvorrichtung auf dem Lenkrad angeordnet, die den Finger vollumfänglich umschließt, wobei auf einer dem Lenkrad abgewandten Innenseite der Messvorrichtung ein lichtemittierender Teil der Messvorrichtung angeordnet ist und auf dieser gegenüberliegend in dem Lenkrad ein lichtempfangener Teil der Messvorrichtung angeordnet ist.

In einem weiteren Beispiel beschreibt die Veröffentlichung EP 1 632 371 A1 eine Lichtremissionsmessungsvorrichtung, die in dem Lenkrad integriert ist, wobei eine auf dem Lenkrad angebrachte Führung zur Positionierung des Fingers angeordnet ist und der lichtemittierende Teil und der lichtempfangene Teil in dem Lenkrad angeordnet sind und über ein Messfenster in dem Lenkrad die Messung durchführen.

Die Veröffentlichung CA 2 517 184 A1 beschreibt einen optischen,Sensor zur Messung von Herzfrequenz und Blutsauerstoffwerten, bei dem der optische Sensor in einen Griff eines Sportgeräts oder in das Lenkrad eines Fahrzeuges integriert werden kann. Der Lichtsensor enthält einen Lichtemitter und einen Lichtdetektor, die beide in dem Griff oder dem Lenkrad integriert sind und wobei der Lichtdetektor das von der Hand reflektierte Licht misst.

Die Veröffentlichung WO 96/13208 A1 bezieht sich auf einen optischen Sensor mit einer Lichtenergiequelle, einem Gehäuse mit einer Unterstützungsfläche für das zu analysierende Material, einer Kammer mit einem Eingang in Form einer an der Gehäuse-Unterstützungsfläche ausgebildeten Öffnung und einem in der Kammer angeordneten Detektor. Ein erster Abschnitt eines optisch streuenden Mediums ist nun derart angeordnet, dass das optisch streuende Medium zwischen dem Detektor und dem Gewebematerial angeordnet ist, wenn die Unterstützungsfläche angrenzend an das Gewebematerial angeordnet ist, wobei das Gewebematerial die Öffnung bedeckt.

Die Veröffentlichung US2006/009685A1 bezieht sich auf eine Vorrichtung und ein Verfahren für nicht-invasive optische Messungen. Die Messvorrichtung weist eine Halteanordnung auf, die wiederum halbzylindrisch ausgebildet ist, um einen Finger aufzunehmen. In der Halteanordnung sind nun ferner Öffnungen vorgesehen, um die optischen Sende- und Empfangselemente aufzunehmen.

Die Veröffentlichung US 2005/023 9075 A1 offenbart die Erfassung einen Herzrale eines Fahrzeugführers, wobei auch Elektroden auf dem Schaltknauf angeordnet sind.

Die Aufgabe der vorliegenden Erfindung besteht darin, eine verbesserte Vorrichtung zum optischen Erfassen von Vitalparametern einer Person in einem Kraftfahrzeug bereitzustellen.

### Zusammenfassung

Die Aufgabe wird durch eine Vorrichtung gemäß Anspruch 1 gelöst.

Ein weiteres Ausführungsbeispiel der vorliegenden Erfindung schafft eine Vorrichtung zum Überwachen zumindest eines Vitalparameters einer Person in einem Kraftfahrzeug mit folgenden Merkmalen: einer Vorrichtung zum Erfassen des zumindest einen Vitalparameters; und einer Vorrichtung zum Auswerten des zumindest einen Vitalparameters, die ausgebildet ist, einen Wert des zumindest einen Vitalparameters mit zumindest einem gegebenen Schwellwert zu vergleichen, und eine Warnmeldung auszugeben, wenn der Wert des zumindest einen Vitalparameters diesen zumindest einen Schwellwert über- oder unterschreitet.

Ausführungsbeispiele der Erfindung basieren auf der Erkenntnis, dass komplizierte und extreme Lichtverhältnisse im Automobil, vor allem am Lenkradkranz, Fremdlichtartefakte verursachen, die die Messung verfälschen können.

Die Erfindung basiert auf der Erkenntnis, dass bekannte Lösungen für die Integration von optischen Sensoren in Automobilumgebungen, z. B. die Integration eines optischen Sensors in das Lenkrad, schwer bzw. gar nicht in den Automobilbedingungen realisierbar sind. Die Gründe hierfür sind vor allem nicht geeignete Integrationsorte und die schlechte Signalqualität der optischen Messung, auf die im Folgenden noch näher eingegangen wird.

Der Lenkradkranz stellt viel zu wenig Platz für die Integration der optischen Sensoren zur Verfügung: Hier kann, z. B. das Fingerbett nur begrenzt implementiert werden. Eine kleine Fläche der Kontaktstelle "Finger-Sensor" führt zu einer nicht optimalen Verteilung des Anpressdrucks und damit zu Verfälschungen oder sogar zum Verlust der Messdaten. Im Gegensatz dazu bietet z. B. der Schaltknauf ausreichenden Platz, um das Fingerbett zu implementieren und ermöglicht somit eine zuverlässige Erfassung der Messdaten.

Zudem ist das Lenkrad eines der am beweglichsten Bedienelemente des Autos bzw. eines der Bedienelemente, das am meisten bewegt wird. Dadurch werden die optischen Messungen einen großen Anteil von Bewegungsartefakten beinhalten, die wiederum die Erfassung der Messdaten bzw. Vitalparameter beeinträchtigen.

Außerdem beeinflussen große Temperaturschwankungen am Lenkradkranz selbst sehr stark die Messwerte.

Ausführungsbeispiele der Vorrichtung zum optischen Erfassen von zumindest einem Vitalparameter weisen ein lichtempfindliches Element, z. B. einen optischen Sensor, auf, der fest in einem Bedienelement des Automobils bzw. Kraftfahrzeugs integriert ist. Es ist keine weitere Ausrüstung notwendig, die am Körper des Fahrers getragen wird, so dass die Beeinträchtigung des Fahrers so gering wie möglich gehalten ist. Bedienelemente des Kraftfahrzeugs sind z. B. der Schaltknauf oder das Lenkrad, umfassen im weiteren Sinne aber auch andere Elemente des Automobils, mit denen der Fahrer aber auch ein Beifahrer in Kontakt kommt, wie z. B. die Mittelarmlehne oder die Seitenarmlehnen.

Ausführungsbeispiele der Vorrichtung zum Erfassen zumindest eines Vitalparameters ermöglichen dank des Fingerbetts in einem Bedienelement des Kraftfahrzeugs eine Reduzierung störender Einflüsse anderer Lichtquellen von außerhalb der optoelektronischen Sensoranordnung auf die Messung bzw. Erfassung. Diese anderen Lichtquellen, unabhängig ob es sich dabei um eine direkte Lichteinstrahlung oder um reflektiertes Licht handelt, werden auch als Fremdlicht bezeichnet.

Ausführungsbeispiele, bei denen das Fingerbett in dem Schaltknauf als Bedienelement integriert ist, ermöglichen zudem eine optimale Verteilung des Anpressdrucks der Fingerkuppe. Durch dieses Fingerbett wird die Fingerkuppe auch möglichst optimal auf dem optischen Sensor platziert, d. h. geführt. Der Schaltknauf bietet deutlich mehr Platz als herkömmliche Lenkräder oder Lenkradkränze, um das Fingerbett und den optischen Sensor (optoelektronische Sensoranordnung) und eventuell auch die elektronische Schaltung zur Steuerung und Auswertung der Daten zu integrieren. Zudem ist der Schaltknauf auch nicht in dem Umfang sicherheitsrelevant wie das Lenkrad. Dies kann zu einer höheren Akzeptanz von Seiten der Industrie für zukünftige technologische Herstellungsprozesse führen.

Bei Ausführungsbeispielen mit einem in den Schaltknauf integrierten Fingerbett werden zudem komplizierte bzw. extreme Lichtverhältnisse im Automobil einerseits durch die etwas tiefere Lage des Schaltknaufs (im Vergleich zum Lenkrad) relativ zur Windschutzscheibe und den Seitenfenstern, eliminiert bzw. reduziert. Ferner wird das lichtempfindliche Element bzw. Photoelement der optoelektronischen Sensoranordnung im Schaltknauf von Fremdlicht durch speziellen Aufbau des Fingerbetts geschützt, das den Finger bündig umschließt.

### Kurzbeschreibung der Figuren

Ausführungsbeispiele der vorliegenden Erfindung werden nachfolgend unter Bezugnahme auf die beiliegenden Zeichnungen näher erläutert.
- Fig. 1: zeigt ein Ausführungsbeispiel einer Vorrichtung zum Erfassen zumindest eines Vitalparameters einer Person in einem Kraftfahrzeug in Form eines in einen Schaltknauf integrierten Lichtremissions-Sensors.
- Fig. 2: zeigt das Ausführungsbeispiel gemäß Fig. 1 mit angelegter Hand der Person, für die der zumindest eine Vitalparameter erfasst werden soll.
- Fig. 3: zeigt ein Ausführungsbeispiel einer in ein Fingerbett eines Bedienelements angeordneten optoelektronischen Sensoranordnung (hier eines Lichtremissions-Sensors).
- Fig. 4: zeigt eine Vorrichtung zum Überwachen zumindest eines Vitalparameters einer Person in einem Kraftfahrzeug und eine durch Schwellwertüberschreitung ausgelöste Datenübertragung an einen Dritten, z.B. Ärzte-PC oder Server.
- Fig. 5 und 6: zeigen zwei Fotos eines Ausführungsbeispiels einer Vorrichtung zum Erfassen zumindest eines Vitalparameters einer Person in Form eines in einen Schaltknauf integrierten Lichtremissions-Sensors.
- Fig. 7: zeigt ein Foto eines weiteren Ausführungsbeispiels einer Vorrichtung zum Erfassen zumindest eines Vitalparameters einer Person in Form eines in einen Schaltknauf integrierten Lichtremissions-Sensors.
- Fig. 8: zeigt ein Ausführungsbeispiel einer Bedienoberfläche für eine ärztliche Anwendung.

Dabei werden in der vorliegenden Erfindung für Objekte und Funktionseinheiten, die gleiche oder ähnliche funktionelle Eigenschaften aufweisen, gleiche Bezugszeichen verwendet.

### Detaillierte Beschreibung

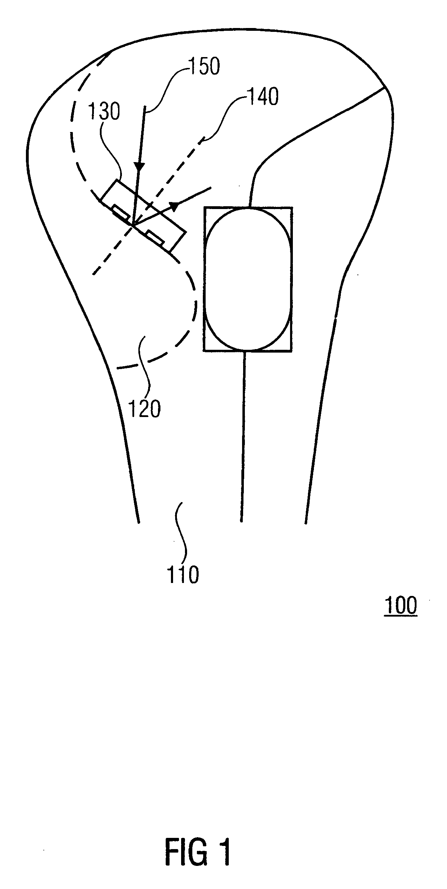

Fig. 1 zeigt ein Ausführungsbeispiel einer Vorrichtung 100 zum Erfassen zumindest eines Vitalparameters einer Person in einem Kraftfahrzeug.

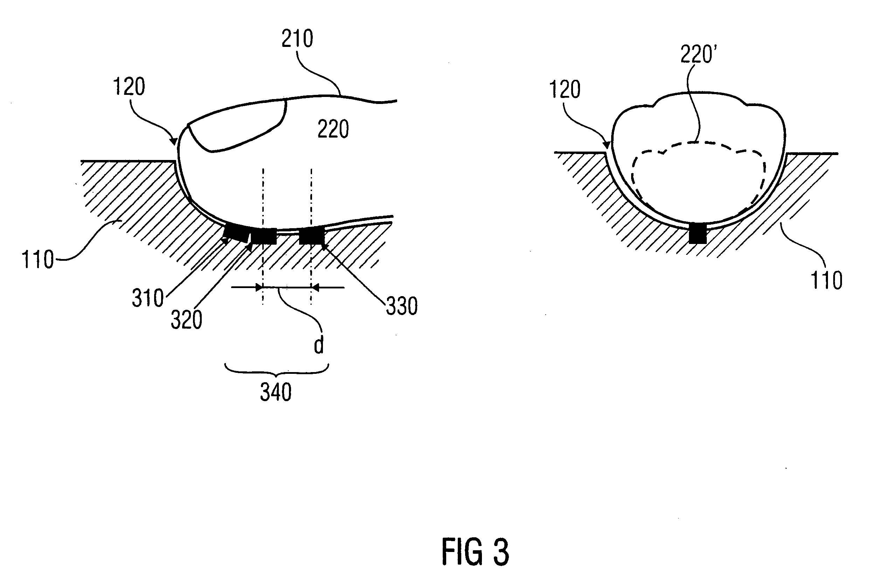

Die Vorrichtung 100 zum Erfassen weist einen Schaltknauf 110 bzw. Knauf 110 eines Schalthebels auf, der ein Fingerbett 120 (siehe gestrichelte Linie) aufweist, wobei in dem Fingerbett 120 ein Lichtremissions-Sensor 130 oder allgemein eine optoelektronische Sensoranordnung 130 angeordnet ist. Der in den Schaltknauf 110 integrierte Lichtremissions-Sensor 130 weist zwei Lichtquellen, eine rote Diode und eine infrarote Diode auf, und einen Photosensor bzw. eine Photodiode, wie dies später anhand von Fig. 3 näher erläutert wird. Die Lichtquellen und der Photosensor liegen in derselben Ebene und befinden sich nahe beieinander. Die Lichtquellen strahlen in das Gewebe des Fingers und der Photosensor misst die reflektierten remittierten Anteile des Lichtfeldes, wie dies schematisch anhand des Lots 140 (gestrichelte Linie senkrecht zu der Ebene des Sensorbereichs bzw. des Bereichs des Fingerbetts 120, in dem die Lichtquellen und der Photosensor angeordnet sind) sowie dem angedeuteten Strahlenverlauf 150 des reflektierten Anteils des Lichtfeldes dargestellt ist.

Der Sensor 130 ist in das Fingerbett 120 integriert, wobei das Fingerbett 120 eine spezielle Vertiefung bzw. Mulde in dem Schaltknauf 110 oder allgemeiner in einem Bedienelement des Kraftfahrzeugs ist, in den ein Finger des Fahrers bequem eingelegt werden kann. Durch dieses Fingerbett 120 wird die Fingerkuppe möglichst optimal auf dem optischen Sensor 130 platziert. Gleichzeitig wird das Photoelement des Sensors 130 vor Außenbeleuchtung geschützt und die angewandte Kraft an der Kontaktstelle zwischen Finger und Sensor 130 gleichmäßig verteilt. Ein Ausführungsbeispiel des Fingerbetts 120 kann dabei eine oder mehrere der folgenden ergonomischen Anforderungen erfüllen:
- bequeme Lage des Fingers,
- optimal verteilter Anpressdruck bei der Auflage des Fingers, z.B. durch Führung des Fingers mittels des Fingerbetts,
- Querlage des Fingers am Schaltknauf möglich, um verschiedene Griffgewohnheiten der Fahrer zu berücksichtigen,
- Abmessungen des Fingerbetts 120 derart, dass unabhängig von der variierenden Fingergröße von Mensch zu Mensch ein bündiges Aufnehmen des Fingers in dem Fingerbett 120 erreicht wird und störende Einflüsse durch Fremdlicht vermieden bzw. zumindest reduziert werden,
- (automatische) Anpassung der Breite des Fingers 120 an den Finger, und
- Sicherstellen, dass der Finger nicht im Fingerbett eingeklemmt werden kann, indem z.B. das Fingerbett oben offen ist bzw. auf der der optoelektronischen Sensoranordnung gegenüberliegenden Seite zumindest teilweise offen ist, und in anderen Ausführungsbeispielen sich zudem nicht nach oben verjüngt.

Eine Anpassung der Breite des Fingerbetts an den Finger kann beispielsweise dadurch erreicht werden, dass der Schaltknauf 110 austauschbar ist, und für verschiedene Fahrer spezifische Schaltknäufe 110 mit einem an die Größe der Hand, mit einem an den oder die Finger und/oder die Griffgewohnheiten des Fahrers angepassten Fingerbett 120 montierbar sind. Dabei gewährleistet, z.B. eine entsprechende Verriegelung, dass der auswechselbare Schaltknauf 110 fest mit dem Schalthebel verbunden ist. Eine automatische Anpassung eines gemeinsamen Schaltknaufs 110 für mehrere Fahrer und damit eine automatische Anpassung der Breite des Fingerbetts an die verschiedenen Finger kann beispielsweise durch Verwendung dehnbarer Materialien im Bereich des Fingerbetts 120 erreicht werden, wobei die jeweilige fahrerspezifische Einstellung der Fingerbettbreite beispielsweise in einem Fahrerprofil neben anderen fahrerspezifischen Einstellungen wie Einstellung der Innen- und Außenspiegel, des Sitzes etc. gespeichert und gesteuert wird.

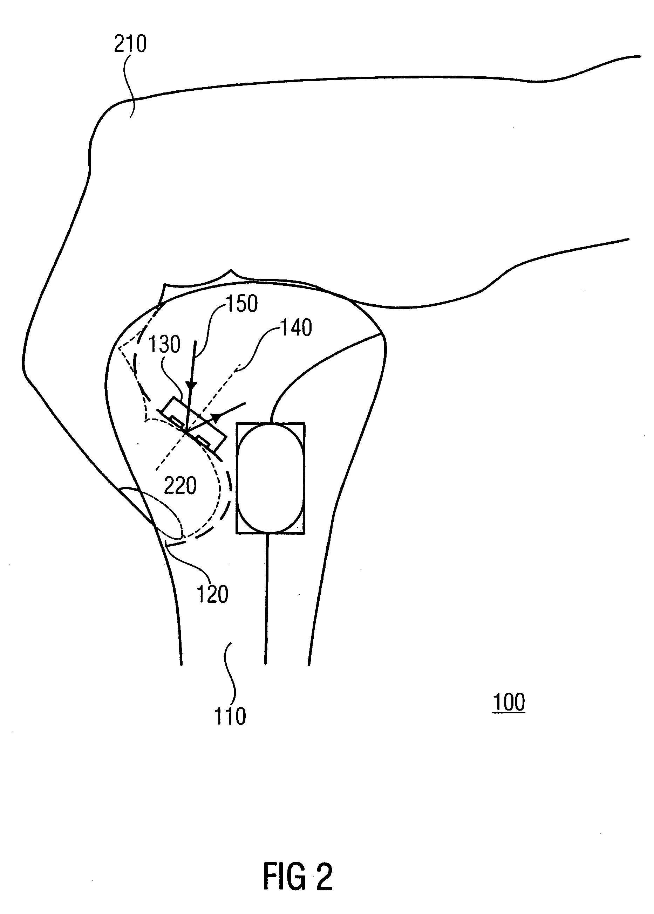

Fig. 2 zeigt das Ausführungsbeispiel der Vorrichtung 100 zum Erfassen zumindest eines Vitalparameters gemäß Fig. 1 mit angelegter Hand 210 und einer an die optoelektronische Sensoranordnung 130 angelegten Fingerkuppe 220.

Fig. 3 zeigt ein Ausführungsbeispiel eines Fingerbetts eines Bedienelements eines Kraftfahrzeugs mit einer in das Fingerbett 120 integrierten optoelektronischen Sensoranordnung 130 in Form eines Lichtremissions-Sensors. Die optoelektronische Sensoranordnung 130 weist eine rote Diode 310, eine infrarote Diode 320 und eine Photodiode 330 auf, die in einem Sensorbereich 340 des Fingerbetts 120 angeordnet sind. Der Abstand d der infraroten Diode 320 und der Photodiode 330 beträgt beispielsweise 8 mm. Wie in Fig. 3 rechts zu sehen ist, sind die rote Diode, die infrarote Diode und die Photodiode in einer Linie angeordnet. In alternativen Ausführungsbeispielen können diese auch anders zueinander angeordnet sein.

Die rote Diode ist beispielsweise ausgebildet, um ein sichtbares Licht im 660nm-Bereich zu erzeugen, und die infrarote Diode 320, um ein Licht im für den Menschen nicht-sichtbaren Wellenlängenbereich von 940 nm zu erzeugen. Durch die unterschiedliche Färbung des mit Sauerstoff gesättigten Hämoglobins entstehen für das durchstrahlende Rotlicht bzw. Infrarotlicht eine unterschiedliche Absorption, die der Photosensor 330 misst, wobei eine Auswerteeinheit beispielsweise mittels eines Vergleichs der Messergebnisse mit einer Referenztabelle die Sauerstoffsättigung des Bluts in den Kapillaren erfassen bzw. bestimmen kann. Neben der Sauerstoffsättigung kann mittels der optoelektronischen Sensoranordnung 130 auch allgemein der Puls bzw. die Pulswelle, Pulsrate, Pulsratenvariabilität bestimmt werden. Für die Bestimmung dieser Pulsparameter oder Pulsinformationen ist jedoch, im Gegensatz zu der Bestimmung der Sauerstoffsättigung, eine einzige Lichtquelle der zwei Lichtquellen ausreichend.

Wie zuvor erläutert, ist das Fingerbett 120 vorzugsweise so ausgebildet, dass es Finger bzw. Fingerkuppen verschiedener Größe so führt bzw. aufnimmt, dass die Fingerkuppe 220 in dem Sensorbereich 340 anliegt, um eine optimale Messung zu ermöglichen. Dies kann beispielsweise, wie in Fig. 3 rechts dargestellt, durch ein konkaves Fingerbett 120 erreicht werden. Zur Illustration ist eine kleinere Fingerkuppe 220' (gestrichelte Linie) in Fig. 3 rechts eingezeichnet. Ein konkaves Fingerbett ermöglicht zudem ein bündiges Abschließen von Fingern verschiedener Größe, um störende Einflüsse durch Fremdlicht zu reduzieren.

Die Fig. 1 - 3 zeigen in anderen Worten allgemein eine Vorrichtung zum Erfassen zumindest eines Vitalparameters einer Person in einem Kraftfahrzeug mit folgenden Merkmalen: einer optoelektronischen Sensoranordnung 130 zum Erfassen des zumindest einen Vitalparameters mittels Lichtremission, wobei die optoelektronische Sensoranordnung eine erste Lichtquelle 310, gegebenenfalls eine zweite Lichtquelle 320 und ein lichtempfindliches Element 330 aufweist; wobei die erste Lichtquelle 310, die zweite Lichtquelle 320 und das lichtempfindliche Element 330 in einem Sensorbereich 340 eines Fingerbetts 120 eines Bedienelements 110 des Kraftfahrzeugs angeordnet sind und wobei das Fingerbett 120 ausgebildet ist, um eine Fingerkuppe 220 der Person in einem Sensorbereich 340 des Fingerbetts 120, in dem die erste Lichtquelle 310, die zweite Lichtquelle 320 und das lichtempfindliche Element 330 angeordnet sind, bündig aufzunehmen.

Dabei weist die optoelektronische Sensoranordnung 130 gemäß den Fig. 1 - 3 einen Lichtremissions-Sensor auf, wobei die rote Diode 310 der ersten Lichtquelle entspricht, die infrarote Diode 320 der zweiten Lichtquelle entspricht und der Photosensor bzw. die Photodiode 330 dem lichtempfindlichen Element entspricht. Allgemein ausgedrückt, ist die erste Lichtquelle ausgebildet, ein Licht einer ersten Wellenlänge zu erzeugen, die zweite Lichtquelle ausgebildet, ein Licht einer zweiten Wellenlänge zu erzeugen, wobei die erste und die zweite Wellenlänge unterschiedlich sind. Ferner ist das Fingerbett eine einseitig offene Vertiefung in dem Bedienelement, die ausgebildet ist, um eine Unterseite der Fingerkuppe 220 bündig aufzunehmen. Die einseitige Offenheit des Fingerbetts 120 hat gegenüber Clipsen oder das Fingerbett vollständig umschließende Anordnungen den Vorteil, dass beispielsweise im Straßenverkehr der Finger jederzeit sofort aus dem Fingerbett wieder entfernt werden kann, ohne hängen zu bleiben. Ferner sind die erste Lichtquelle 310, die zweite Lichtquelle 320 und das lichtempfindliche Element 330 in Bezug auf den Finger auf derselben Seite des Fingers angeordnet, und das lichtempfindliche Element 330 ausgebildet, das reflektierte remittierende Licht der ersten und zweiten Lichtquelle 310, 320 zu empfangen.

Gemäß den Fig. 1 und 2 ist das Bedienelement, in dem das Fingerbett 120 integriert ist, ein Schaltknauf.

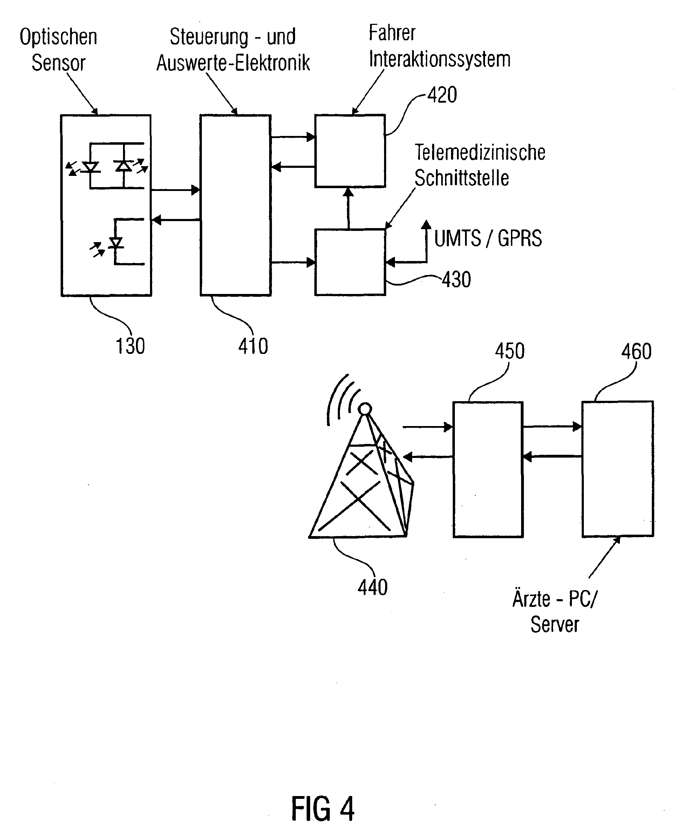

Fig. 4 zeigt ein Ausführungsbeispiel eines Messsystems zur Detektion von Pulswellen, Pulsrate, Pulsratenvariabilität und Sauerstoffsättigung des Blutes einer Person in einem Kraftfahrzeug. Das Messsystem besteht aus einem optischen Sensor 130, einer Steuerungs- und Auswerteelektronik 410, einem Fahrerinteraktionssystem 420, einer telemedizinischen Schnittstelle 430 und einem Ärzte-PC (Personal-Computer) 460. Dabei ist der optische Sensor 130, wie zuvor anhand der Fig. 1 - 3 erläutert, in einem Fingerbett in einem Bedienelement des Kraftfahrzeugs angeordnet und die Steuerungs- und Auswerteelektronik 410, das Fahrerinteraktionssystem 420 und die telemedizinische Schnittstelle 430 ebenfalls in dem Kraftfahrzeug integriert.

In einem weiteren Ausführungsbeispiel kann beispielsweise auch die Steuerungs- und Auswerteelektronik 410 in demselben Bedienelement wie der optische Sensor 130 integriert sein. Der Ärzte-PC oder entsprechende Server ist beispielsweise bei einem Arzt, einer Klinik oder einem anderen Ort aufgestellt und beispielsweise mittels eines Mobilfunknetzes 450, im Englischen auch als Mobile Core Network bezeichnet, und einer Basisstation 440 mit einer mobilen Sende-/Empfangseinheit, die in der telemedizinischen Schnittstelle 330 integriert ist, miteinander verbunden. Das Mobilfunknetz kann beispielsweise ein UMTS-Mobilfunknetz sein (Universal Mobile Telecommunication Standard) oder auf einem anderen Mobilfunkstandard, wie z. B. GPRS (Global Packet Radio System) oder GSM (Global System for Mobile Communication), oder einem anderen Funkkommunikationsnetzwerk, z. B. WLAN (Wireless Local Area Network), basieren.

Die erfassten Vitalparameter werden durch die Auswerteelektronik 410 beispielsweise in drei Warngruppen unterteilt (siehe Tabelle). Für jede der Warngruppen werden der obere und der untere Grenzwert der entsprechenden Vitalparameter definiert. Es werden auch die Warnstufen anhand des Gefährdungsgrades des Fahrers für jede Warngruppe definiert. Bei Überschreiten des gegebenen Grenzwerts der Vitalparameter wird die entsprechende Warnmeldung ausgelöst.

**Tabelle. Definition der Warnstufen.**

| | |
|---|---|
| N / Datenfehler | Die erste Warnstufe entspricht dem normalen Zustand des Fahrers (der Vitalparameter liegt in normalen physiologischen Grenzen). |
| | |
| | Die nicht-auswertbaren bzw. verlorenen Sensordaten werden auch als erste Warnstufe codiert. |
| Achtung | Die zweite Warnstufe entspricht dem Fahrerzustand, wenn auf die Werte der Vitalparameter aufmerksam gemacht werden muss. |
| | |
| | Es folgt die Warnmeldung über die überschrittenen Grenzwerte. |
| | |
| | Es wird dem Fahrer geboten, sich aus dem Verkehr zurückzuziehen. |
| Kritisch | Die dritte Warnstufe entspricht dem Fahrerzustand, wenn eine ärztliche Betreuung notwendig ist. |
| | |
| | Es folgt die Warnmeldung über die überschrittenen Grenzwerte. |
| | |
| | Der Fahrer muss sich aus dem Verkehr zurückziehen. |

In anderen Worten, ein weiteres Ausführungsbeispiel der vorliegenden Erfindung schafft eine Vorrichtung zum Überwachen zumindest eines Vitalparameters einer Person in einem Kraftfahrzeug mit folgenden Merkmalen: einer Vorrichtung 100 zum Erfassen des zumindest einen Vitalparameters, wie sie anhand der Fig. 1 - 3 beschrieben wurde; und zumindest einer Vorrichtung 410 zum Auswerten des zumindest einen Vitalparameters, die ausgebildet ist, einen Wert des zumindest eines Vitalparameters mit zumindest einem gegebenen Schwellwert zu vergleichen und eine Warnmeldung auszugeben, wenn der Wert des zumindest eines Vitalparameters diesen zumindest einen Schwellwert über- oder unterschreitet.

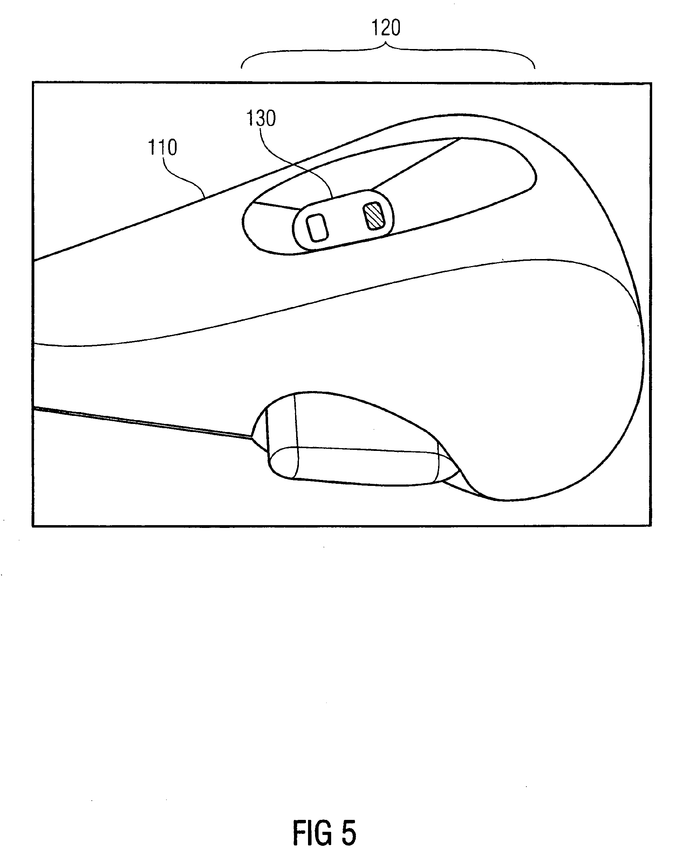

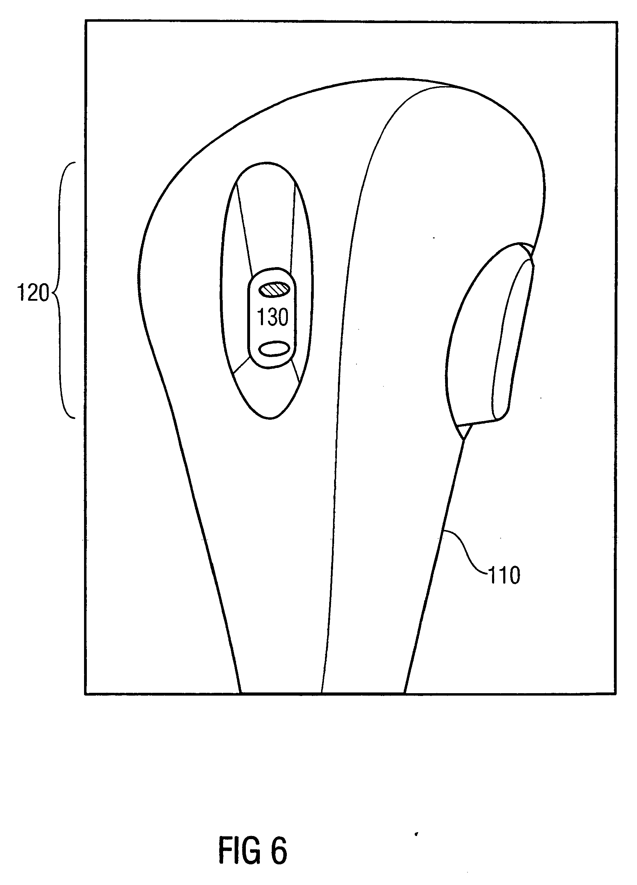

Die Fig. 5 und 6 zeigen eine beispielhafte Integration eines optischen Sensors 130 in ein Fingerbett 120, das wiederum in einen Schaltknauf 110 integriert ist.

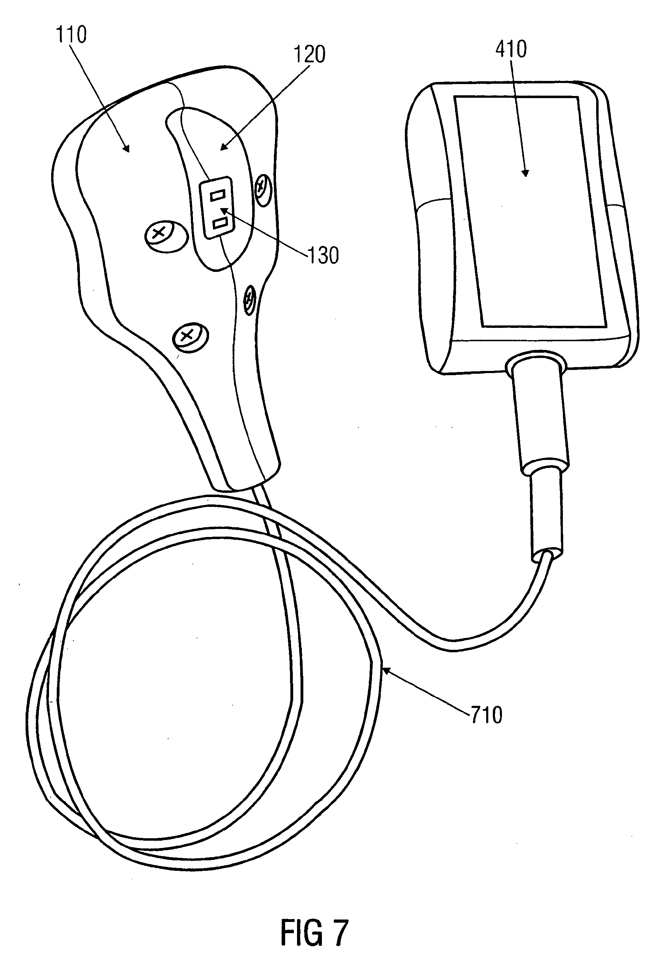

Fig. 7 zeigt einen Prototypen eines im Fingerbett 120 eines Schaltknaufs 110 integrierten optischen Sensors 130, der über ein mehradriges geschirmtes Kabel 710 mit einer Steuerungs- und Auswerteelektronik 410 verbunden ist. Die Steuerungs- und Auswerteelektronik 410 ist über eine in die elektronischen Schaltung der Steuerungs- und Auswerteelektronik 410 integrierte Bluetooth-Schnittstelle mit einer Zentraleinheit, z. B. dem Fahrerinteraktionssystem 420 und der telemedizinischen Schnittstelle 430 (siehe Fig. 4), gekoppelt.

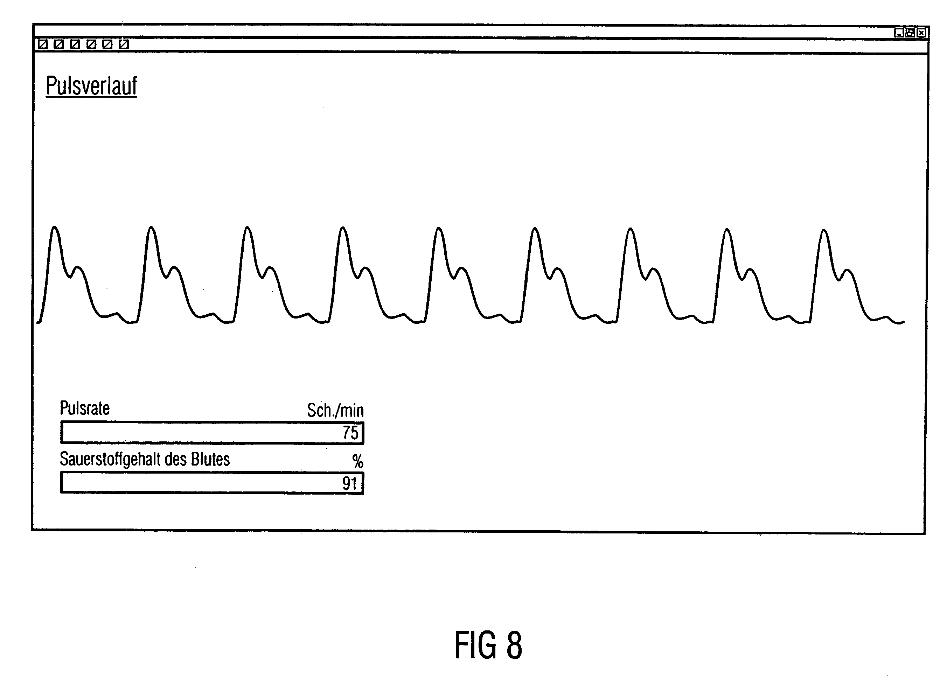

Fig. 8 zeigt ein Beispiel einer Bedienoberfläche für eine ärztliche Anwendung, wie sie beispielsweise auf dem Ärzte-PC 460 ausgeführt werden kann, die, wie in Fig. 8 dargestellt, beispielsweise den Pulsverlauf über die Zeit sowie die Pulsrate, z.B. 75 Schläge pro Minute (siehe Fig. 8), sowie den Sauerstoffgehalt des Blutes, z.B. 91 % (siehe Fig. 8), anzeigt.

Bezug nehmend auf die vorhergehenden Erläuterungen kann daher gesagt werden, dass Ausführungsbeispiele der vorliegenden Erfindung für ein Verfahren und Messsystem zur optischen Detektion der Pulswelle, Pulsrate, Pulsratenvariabilität und Sauerstoffsättigung des Blutes eines Fahrers im Schaltknauf eines Kraftfahrzeugs, ein Messsystem zur Detektion der Pulswelle, Pulsrate, Pulsratenvariabilität und Sauerstoffsättigung des Blutes eines Fahrers eines Kraftfahrzeugs mittels im Schaltknauf integriertem optischen Sensor und/oder einen optischen Sensor zur Detektion der Pulswelle, Pulsrate, Pulsratenvariabilität und Sauerstoffsättigung des Blutes eines Fahrers im Schaltknauf anwendbar sind. Dabei können Ausführungsbeispiele der vorliegenden Erfindung sowohl als medizinisches System zum Überwachen, im Englischen auch als "Monitoring" bezeichnet, der Vitalparameter eines Menschen, insbesondere der Detektion der Pulswelle, der Pulsrate, der Pulsratenvariabilität und der Sauerstoffsättigung des Blutes eingesetzt werden, als auch als eine Einrichtung zum Insassenschutz bzw. zur Insassenwarnung und Fahrerunterstützung eingesetzt werden. Dabei liegt das Anwendungsgebiet der Erfindung beispielsweise im Bereich präventiver, überwachender und begleitender Medizin für den Einsatz im Fahrzeug, Stichwort "Fahrerassistenzsysteme", unter bzw. ohne telemetrische ärztliche Aufsicht. Dabei können Ausführungsbeispiele der vorliegenden Erfindung eingesetzt werden, um im Alltag Fahrer mit kardiologischen Risikofaktoren beim Autofahren in Bezug auf Müdigkeit, Aufmerksamkeit, Stress zu überwachen.

Dabei schaffen Ausführungsbeispiele der vorliegenden Erfindung ferner ein Messsystem, das eine Durchführung der Messungen der Pulswellenparameter, der Pulsrate, der Pulsratenvariabilität und der Sauerstoffsättigung des Blutes unter Automobilbedingungen mit geringer Beeinträchtigung des Fahrers ermöglicht. Dabei werden die Messwerte dem Fahrer beispielsweise via Fahrerinteraktionssystem sowie beispielsweise einem betreuenden Arzt via telemetrischer Schnittstelle zugänglich gemacht. Die erfassten Werte können klassifiziert und mit einem persönlichen Fahrerprofil abgeglichen werden. Beim Überschreiten der Grenzwerte wird beispielsweise eine Warnmeldung bzw. ein Alarm oder ein Notdienstaufruf ausgelöst.

Ausführungsbeispiele der vorliegenden Erfindung betreffen ferner eine Vorrichtung mit einem optischen Sensor im Schaltknauf eines Kraftfahrzeugs zur Detektion der Pulswelle, bei dem unterschiedliche elektromagnetische Wellenlängen im Bereich des nicht sichtbaren infraroten und des sichtbaren roten elektromagnetischen Spektrums vorgesehen sind.

Ferner schaffen Ausführungsbeispiele der vorliegenden Erfindung eine Vorrichtung zum Überwachen einer Pulsrate, Pulsratenvariabilität und Sauerstoffsättigung des Blutes des Fahrers im Automobil basierend auf der erfassten Pulswelle und/oder ein Messsystem zur Erfassung der Pulswelle, Pulsrate, Pulsratenvariabilität und Sauerstoffsättigung des Blutes des Fahrers im Automobil.

Ferner schaffen Ausführungsbeispiele der vorliegenden Erfindung ein Fahrerassistenzsystem zur medizinischen Überwachung des Gesundheitszustandes des Fahrers, insbesondere des Pulswellenprofils, der Pulsrate, der Pulsratenvariabilität und Sauerstoffsättigung des Blutes. Dabei ergebne sich weitere Anwendungsfelder, z.B. durch eine Verkopplung von Einflüssen auf den Fahrer, wie z.B. Stress, mit einer entsprechenden Assistenzleistung, beispielsweise einer entsprechenden Aufforderung mittels einer Ansage oder Anzeige gegenüber dem Fahrer, die Geschwindigkeit zu reduzieren.

## Patentansprüche

1. Vorrichtung (100) zum Erfassen zumindest eines Vitalparameters einer Person in einem Kraftfahrzeug mit folgenden Merkmalen:
einer optoelektronischen Sensoranordnung (130) zum Erfassen des zumindest einen Vitalparameters mittels Lichtremission, wobei die optoelektronische Sensoranordnung zumindest eine erste Lichtquelle (310) und ein lichtempfindliches Element (330) aufweist, wobei die erste Lichtquelle und das lichtempfindliche Element in einem Fingerbett (120) eines Bedienelements (110) des Kraftfahrzeugs angeordnet sind, wobei das Fingerbett (120) ausgebildet ist, um eine Fingerkuppe (220) der Person in einem Sensorbereich (340) des Fingerbetts (120), in dem die erste Lichtquelle (310) und das lichtempfindliche Element (330) angeordnet sind, bündig aufzunehmen, wobei das Fingerbett (120) eine einseitig offene Vertiefung in dem Bedienelement (110) ist, und wobei die einseitig offene Vertiefung ausgebildet ist, um eine Unterseite der Fingerkuppe (220) bündig aufzunehmen, wobei das Bedienelement (110) ein Knauf eines Schalthebels ist.

2. Vorrichtung nach Anspruch 1, wobei die erste Lichtquelle (310) und das lichtempfindliche Element (330) in einer gleichen Ebene des Fingerbetts (120) angeordnet sind.

3. Vorrichtung nach einem der Ansprüche 1 oder 2, wobei der Vitalparameter eine Pulsinformation ist.

4. Vorrichtung nach einem der Ansprüche 1 bis 3, wobei die optoelektronische Sensoranordnung (130) zusätzlich eine zweite Lichtquelle (320) aufweist, die in dem Fingerbett (120) des Bedienelements (110) angeordnet ist.

5. Vorrichtung nach Anspruch 4, wobei der Vitalparameter eine Sauerstoffsättigung des Bluts der Person ist.

6. Vorrichtung zum Überwachen zumindest eines Vitalparameters einer Person in einem Kraftfahrzeug mit folgenden Merkmalen:
einer Vorrichtung (100) zum Erfassen des zumindest einen Vitalparameters gemäß einem der Ansprüche 1 bis 5; und
einer Vorrichtung (410) zum Auswerten des zumindest einen Vitalparameters, die ausgebildet ist, einen Wert des zumindest einen Vitalparameters mit zumindest einem gegebenen Schwellwert zu vergleichen und eine Warnmeldung auszugeben, wenn der Wert des zumindest einen Vitalparameters diesen zumindest einen Schwellwert über- oder unterschreitet.

7. Vorrichtung nach Anspruch 6, mit folgendem Merkmal:
einem Fahrerassistenzsystem;
wobei die Vorrichtung zum Auswerten (410) ausgebildet ist, anhand des Über- und Unterschreitens des zumindest einen Schwellwertes kardiologische Risikofaktoren oder eine chronische Lungenerkrankung der Person zu überwachen, Müdigkeit und/oder Stress der Person zu erkennen, und das Fahrerassistenzsystem ausgebildet ist, bei Über- und Unterschreiten des zumindest einen Schwellwertes der Person abhängig davon eine Assistenzleistung anzubieten oder zu erbringen.

## Claims

1. A device (100) for detecting at least one vital parameter of a person in a vehicle, comprising:
an optoelectronic sensor arrangement (130) for detecting the at least one vital parameter by means of light remission, wherein the optoelectronic sensor arrangement comprises at least one light source (310) and a light-sensitive element (330), wherein the first light source and the light-sensitive element are arranged in a finger bed (120) of an operating element (110) of the vehicle, wherein the finger bed (120) is implemented to integrate a fingertip (220) of the person flush in a sensor area (340) of the finger bed (120) in which the first light source (310) and the light sensitive element (330) are arranged, wherein the finger bed (120) is a recess open to one side in the operating element (110), and wherein the recess open to one side is implemented to receive a bottom side of the fingertip (220) flush, wherein the operating element (110) is a knob of a gear lever.

2. The device according to claim 1, wherein the first light source (310) and the light-sensitive element (330) are arranged in the same plane of the finger bed (120).

3. The device according to one of claims 1 or 2, wherein the vital parameter is a pulse information.

4. The device according to one of claims 1 to 3, wherein the optoelectronic sensor arrangement (130) additionally comprises a second light source (320) arranged within the finger bed (120) of the operating element (110).

5. The device according to claim 4, wherein the vital parameter is an oxygen saturation of the blood of the person.

6. A device for monitoring at least one vital parameter of a person in a vehicle, comprising:
a device (100) for detecting the at least one vital parameter according to one of claims 1 to 5; and
a device (410) for evaluating the at least one vital parameter which is implemented to compare a value of the at least one vital parameter to at least one given threshold value and to output a warning message when the value of the at least one vital parameter exceeds or falls below this at least one threshold value.

7. The device according to claim 6, comprising:
a driver assistance system;
wherein the device for evaluating (410) is implemented, by means of exceeding or falling below the at least one threshold value, to detect cardiologic risk factors or a chronic lung disease of a person, detect fatigue and/or stress of the person, and the driver assistance system is implemented, when exceeding or falling below the at least one threshold value of the person, to offer or execute an assistance service depending thereon.

## Revendications

1. Dispositif (100) pour détecter au moins un paramètre vital d'une personne dans un véhicule motorisé, aux caractéristiques suivantes:
un aménagement de capteur optoélectronique (130) destiné à détecter l'au moins un paramètre vital à l'aide d'une rémission de lumière, l'aménagement de capteur optoélectronique présentant au moins une source de lumière (310) et un élément sensible à la lumière (330), la première source de lumière et l'élément sensible à la lumière étant disposés dans un lit à doigt (120) d'un élément de commande (110) du véhicule motorisé, le lit à doigt (120) étant réalisé pour recevoir en liaison de forme le doigt (220) de la personne dans une zone de capteur (340) du lit à doigt (120) dans laquelle sont disposés la première source de lumière (310) et l'élément sensible à la lumière (330), le lit à doigt (120) étant un creux ouvert d'un seul côté dans l'élément de commande (110), et le creux ouvert d'un seul côté étant réalisé pour recevoir en liaison de forme un côté inférieur du doigt (220), l'élément de commande (110) étant un bouton d'un levier de commande.

2. Dispositif selon la revendication 1, dans lequel la première source de lumière (310) et l'élément sensible à la lumière (330) sont disposés dans un même plan du lit à doigt (120).

3. Dispositif selon l'une des revendications 1 ou 2, dans lequel le paramètre vital est une information de pouls.

4. Dispositif selon l'une des revendications 1 à 3, dans lequel l'aménagement de capteur optoélectronique (130) présente en outre une deuxième source de lumière (320) qui est disposée dans le lit à doigt (120) de l'élément de commande (110).

5. Dispositif selon la revendication 4, dans lequel le paramètre vital est une saturation d'oxygène du sang de la personne.

6. Dispositif pour surveiller au moins un paramètre vital d'une personne dans un véhicule motorisé, aux caractéristiques suivantes:
un dispositif (100) destiné à détecter l'au moins un paramètre vital selon l'une des revendications 1 à 5; et
un dispositif (410) destiné à évaluer l'au moins un paramètre vital, lequel est réalisé pour comparer une valeur d'au moins un paramètre vital avec au moins une valeur de seuil donnée et à sortir un avertissement lorsque la valeur de l'au moins un paramètre vital excède ou descend au-dessous de cette au moins une valeur de seuil.

7. Dispositif selon la revendication 6, à la caractéristique suivante:
un système d'assistance au conducteur;
le dispositif destiné à évaluer (410) étant réalisé pour surveiller, à l'aide du dépassement et de la descente au-dessous de l'au moins une valeur de seuil, les facteurs de risque cardiologique ou une maladie pulmonaire chronique de la personne, identifier la fatigue et/ou le stress de la personne, et le système d'assistance au conducteur est réalisé pour offrir ou apporter à la personne une prestation d'assistance, en cas de dépassement ou de descente au-dessous de l'au moins une valeur de seuil, indépendamment de ce dernier.
